Europäisches Patentamt

European Patent Office

Office européen des brevets

(11) Publication number : **0 514 015 A1**

## (12) EUROPEAN PATENT APPLICATION

(21) Application number : 92303458.1

(22) Date of filing : 16.04.92

(51) Int. Cl.⁵ : **A61K 31/00,** A61K 31/44,
A61K 31/565

(30) Priority : 19.04.91 US 688656

(43) Date of publication of application :
19.11.92 Bulletin 92/47

(84) Designated Contracting States :
CH DE FR GB IT LI NL

(71) Applicant : MERCK & CO. INC.
126, East Lincoln Avenue P.O. Box 2000
Rahway New Jersey 07065-0900 (US)

(72) Inventor : Elbrecht, Alex
65 Maple Street
Watchung, NJ 07060 (US)
Inventor : Smith, Roy G.
528 Forest Avenue
Westfield, NJ 07090 (US)

(74) Representative : Thompson, John Dr. et al
Merck & Co., Inc. European Patent
Department Terlings Park Eastwick Road
Harlow, Essex CM20 2QR (GB)

(54) Control of sex differentiation in fish.

(57)   Fish or shellfish eggs, fertilized embryos or hatched fry or shellfish are treated with steroid biosynthesis inhibitors or antagonists which prevents the conversion of androgens to estrogens. By blocking the production of estrogens the genotypic female is converted into a phenotypic male. The phenotypic conversion of females to males gives the treated fish the advantage of male growth characteristics. Such treatment will also allow the production of monosex populations of fish which can be used to limit reproduction and control overpopulation of specific aquatic environments. Steroid biosynthesis inhibitors and/or antagonists will also induce smoltification in salmon species.

EP 0 514 015 A1

## BACKGROUND OF THE INVENTION

Males of commercially important fish species gain weight faster than do females of the same species. This weight gain, generally resulting from feed efficiency, advantage allows male fish to reach a marketable weight before females. A uniform weight gain of both male and female aquaculture reared fish would have a significantly positive economic impact on production for all important fish species.

Phenotypic female-to-male and male-to-female sex-reversal has been achieved in several different species of fish. The process usually involves treatment of eggs with sex steroids or feeding sex steroids to the fry. Sex-reversal was caused in largemouth bass by feeding 5-week-old fish for six weeks with feed soaked in steroids or brine shrimp raised in tanks containing steroids, Garret, Prog. Fish-Cult. 51: 146-148 (1989). Both methods were used with testosterone and androsterone(100mg/kg feed) to masculinize females. Similarly, in carp, *Cyprinus carpio*, oral administration of 17-alpha-methyltestosterone at 50ppm in feed from weeks 6 through 11 after hatching, produces stocks consisting of greater than 90% phenotypic males, Komen et al, Aquaculture 78: 349-363 (1989). For tilapia, *Oreochromis aureus,* a broad range of doses of either 17-alpha-ethynyltestosterone or 17-alpha-methyltestosterone is effective for sex-reversing when fed to 3 to 5-day old fry for a period of 22 days, McGeachin et al, Aquaculture 61: 317-321 (1987). This suggests that overdosing does not interfere in the production of monosex males. Piferrer and Donaldson, Aquaculture 77: 251-262 (1989), identified the labile period for controlling sexual differentiation by hormonal treatment in coho salmon, *Oncorhynchus kisutch*. Best results for phenotypic conversion to males were obtained by immersion of 6-day-old fry for 2 hours in water containing 400 micrograms 17-alpa-methyltestosterone per liter.

The process of sexual differentiation in fish is not well understood. Chromosomal sex determination varies from one species to another. Results largely obtained from sex reversal experiments suggest that both female catfish, *Ictalurus punctatus,* Davis et al Gen. Comp. Endocrinol. 78: 218-223 (1990), and female tilapia, *Oreochromis niloticus*, Scott et al, Aquaculture 78:237-251(1989), are homogametic (XX). The period of sexual differentiation also varies from one species to another. In carp, *Cypripnus carpio,* this starts 7 to 9 weeks after hatching with mitotic proliferation of germ cells. Male and female gonads can be distinguished histologically at 17 weeks, Komen et al, Aquaculture 78:349-363(1989). In coho salmon, *Oncorhynchus kisutch*, sexual differentiation was observed to occur at 27 days post-hatching, Piferrer and Donaldson, Aquaculture 77: 251-262 (1989).

The role of sex steroids in the process of sexual differentiation is also riot well understood although the steroidogenic capability during sexual differentiation of some species has been measured, Fitzpatrick et al, Proc. Third Int. Symp. Reprod. Physiol. Fish, St. John's, Newfoundland, 2-7 August, 1987. Others have proposed various models based on androgens and estrogens to act as sex specific determinants, Yamamoto, In: W.S. Hoar and D.J. Randall (editors), Fish Physiology, Vol. III, Reproduction, Academic Press, New York, NY, Chap. 3, pp. 117-175, 1969. As a general rule, it seems that to achieve sex-reversal, it is necessary to treat fry before and also probably during the period of sexual differentiation with the appropriate sex steroid.

## OBJECTS OF THE INVENTION

It is, accordingly, an objective of the present invention to provide a means of converting genotypic female fish into phenotypic male fish. Another object is to treat fish or shellfish eggs, embryos or fry with steroid biosynthesis inhibitors or antagonists to prevent the conversion of testosterone to estradiol. A further object is to treat fish or shellfish eggs, embryos or fry with an aromatase inhibitor to convert female genotypic fish into male phenotypic fish. Another object is to convert genotypic female fish into phenotypic male fish so that following sexual development all treated fish will gain weight at the rate of males or that the converted males will gain weight at a rate greater than non-treated females. A further object is to obtain phenotypically male fish with a female genotype. Aother object is to induce smoltification of salmon species.

## SUMMARY OF THE INVENTION

Fish or shellfish eggs, fertilized embryos are treated with steroid biosynthesis inhibitors or antagonists which prevents the conversion of androgens to estrogens. By blocking the production of estrogens the genotypic female is converted into a phenotypic male. The phenotypic conversion of females to males gives the treated fish the advantage of male growth characteristics. Such treatment will also allow the production of monosex populations of fish which can be used to limit reproduction and control overpopulation of specific aquatic environments. Steroid biosynthesis inhibitors and/or antagonists will also induce smoltification in salmon species.

## DETAILED DESCRIPTION OF THE INVENTION

The present invention relates to the conversion of a genotypic female fish or shellfish to a phenotypic male fish or shellfish. Fish is defined herein as any fish, salt or fresh water, warm or cold water, that serve as a source of meat and that can be cultivated under aquaculture conditions. Fish include, but are not limited to, commercially important fish such as catfish , salmon, trout, bass, carp, coho salmon, pike, zebra fish, loach, medaka, wuchang fish and tilapia. Shellfish is defined herein as any crustacea, including but not limited to, lobsters, shrimp, prawns, crawfish and crabs and any crustacea that can be cultivated under aquaculture conditions. Shellfish is further defined as any molluscs, including but not limited to, oysters, mussels, scallops, clams and any molluscs that can be cultivated under aquaculture conditions. Eggs, fertilized embryos or fry of any fish or shellfish capable of being reared by aquaculture are treated with an aromatase inhibitor to prevent the conversion of testosterone to estradiol. Aromatase is an enzyme complex incorporating a NADPH-cytochrome c-reductase and a cytochrome $P_{450}$ component which mediates the conversion of androgens to estrogens, Bellino, J. Steroid Biochem. 17: 261-270 (1982). The reaction is believed to involve three hydroxylation steps, two at the C-19 position (Meyer, Biochem. Biophys. Acta 17: 441-442 [1955]; Morato et al., Biochem. Biophys. Res. Comm. 6: 334-338 [1961]) and one at C-2 (Hahn and Fishman, J. Biol. Chem. 259: 1689-1694 [1984]; Brodie et al., J. Am. Chem. Soc. 91: 1241-1242 [1969]) which result in the conversion of the A ring of the androgen molecule to an aromatic ring. Since aromatization is a unique reaction in the biosynthesis of steroids, specific inhibitors should not cause deprivation of other essential steroids. The inhibition or blocking of the conversion of androgens (testosterone, androstenedione) to estrogens (estradiol, estrone) results in an accumulation of androgens (testosterone, androstenedione) which may allow the gonad in genetic females to differentiate into a testis resulting in the production of phenotypic male fish from genotypic female fish. An aromatase inhibitor as defined herein is any steroidal or non-steroidal compound which prevents the conversion of androgens to estrogens. The compounds include substrate analogues of androstenedione, testosterone or other steroidal substances involved in the aromatase pathway, Henderson, J. Steroid Biochem. 27: 905-914 (1987). Non-steroidal compounds that block aromatase activity are also included within the scope of the invention. These non-steroidal compounds include compounds or analogues that can bind to the enzymatic active site of aromatase and inhibit enzymatic activity. Non-steroidal or steroidal compounds will also include compounds or analogues that bind to the enzyme at a site away from the enzymatic site and cause a structural change in the enzyme which results in a loss of enzymatic activity. Aromatase inhibitors further include non-steroidal compounds that interfere with cytochrome $P_{450}$ mediated hydroxylations such as those described by Brodie et al., J. Steroid Biochem. 27: 899-903 (1987).

The following compounds are known aromatase inhibitors as disclosed by the associated reference. The reference will also directly contain a method for making the compound or will direct one who wishes to use the compound to a method for producing the compound. The aromatase inhibitors of the instant invention include, but are not limited to, the following compounds: 6-[(1H-imidazol-1-yl)phenylmethyl]-1 methyl-1H-benzotriazole, 6-[(4-chlorophenyl)(1H-1,2,4-triazol-1-yl)methyl]-1-methyl-1H-benzotriazole as described in Publication No. 293,978; 2,2-[5-(1H-1,2,4,-triazol-1-ylmethyl)-1,3-phenylene]di(2-methylpropiononitrile), 2,2-[5-(imidazol- 1-ylmethyl)- 1,3-phenylene]-di(2-methylpropiononitrile), 2-[3-(1-hydroxy-1-methylethyl)-5-(1H-1,2,4-triazol-1-ylmethylphenyl]-2-methylpropiononitrile, 2,2-[5-dideuterio(1H-1,2,4-triazol-1-yl)methyl- 1,3-phenylene]di(2trideuteriomethyl-3,3,3-(trideuteriopropiononitrile), and 2,2-[5-dideuterio(1H-1,2,4-triazol-1-ylmethyl- 1,3-phenylene)di(2-methylpropiononitrile) as disclosed in European Patent Application, Publication No. 296,749; 1,1-dimethyl-8-(1H-1,2,4-triazol-1-ylmethyl)2(1H)-naphtho[2,1-b] furanone, 1,2-dihydro-1,1-dimethyl-2-oxo-8-(1H-1,2,4-triazol-1-ylmethyl)naphtho-[2,1-b]furan-7-carbonitrile, 1,2-dihydro-1,1-dimethyl-2-oxo-8-(1H-1,2,4-triazol-1-ylmethyl)-naphtho[2,1-b]furan-7-carboxamide, and 1,2-dihydro-1,1-dimethyl-2-oxo-8-[di(1H-1,2,4-triazol-1 yl)methyl]naphtho[2,1-b]furan-7-carbonitrile as disclosed in European Patnet Application, Publication No. 316,097; 2-(4-chlorobenzyl)-2-fluoro-1,3-di(1,2,4-triazol-1-yl)propane, 2-fluoro-2(2-fluoro-4-chlorobenzyl)-1,3-di(1,2,4-triazol-1-yl)propane, 2-fluoro-2-(2-fluoro-4-trifluoromethylbenzyl)-1,3-di(1,2,4-triazol-1-yl)propane, 3-(4-chlorophenyl)-1-(1,2,4-triazol-1-yl)-2-(1,2,4-triazol-1-ylmethyl)butan-2-ol, 2-(4-chloro-α-fluorobenzyl)1,3-di(1,2,4-triazol-1-yl)propan-2-ol, 2-(4-chlorobenzyl)1,3-bis(1,2,4-triazol-1-yl)propane, 4-[2-(4-chlorophenyl)-1,3-di(1,2,4-triazol-1-ylmethyl)ethoxymethyl]-benzonitrile, 1-(4-fluorobenzyl)-2-(2-fluoro-4-trifluoromethylphenyl)-1,3-di(1,2,4-triazol-1-yl)propan-2-ol, 2-(4-chlorophenyl)-1-(4-fluorophenoxy)-1,3-di(1,2,4-triazol-1-yl)propan-2-ol, 1-(4-cyanobenzyl)2-(2,4-difluorophenyl)-1,3-di(1,2,4-triazol-1-yl)-propan-2-ol and 2-(4-chlorophenyl)1-phenyl-1,3-di(1,2,4-triazol-1-yl)-propan-2-ol as described in European Patent Application, Publication No. 299,684; 5-bis(4-chlorophenyl)methylpyrimidine as disclosed in U.S. Patent No. 4,762,836; α,α-bis(4-chlorophenyl)-2-pyrazinemethanol as described in U.S. Patent No. 4,764,376; N-(2,4-difluorophenyl)-N-benzyl-3-pyridinemethanamine and N-(2-chlorophenyl-α-(4-fluorophenyl)-3-pyridinemethanamine as disclosed in U.S. Patent No. 4,744,251; 1-(10,11-dihydro-5H-dibenzo[a,d]cyclohepten-5-yl)-1H-imidazole and 1-(9H-fluoren-9-yl)-1H-imidazole as disclosed in U.S. Patent No. 4,757,082; 3-bis(4-chlorophenyl)-3-methylpyridine and α,α-bis(4-chlorophenyl)-3-pyridinemethanol as disclosed in U.S. Patnet No. 4,757,076; 5H-5-(4-cyanophenyl)-6,7-dihydropyrrolo[1,2-c]imidazole and 5H-5-(4-cyanophenyl)-6,7,8,9-tetrahydroimidazo[1,5-a]azepine and disclosed in U.S. Patent No. 4,728,645; 5-[(1 H-imidazol-1-yl)phenylmethyl]-2-methyl-1 H-benzimidazole and 5-[(3-chlorophenyl)(1 H-imidazol-1-yl)-methyl]-1 H-benzimidazole as disclosed in European Patent Application, Publication No. 260,744; (Z)-α-(1,2,4-triazol-1-ylmethyl)stilbene-4,4'-dicarbonitrile, (Z)-4'-chloro-α-(1,2,4-triazol-1-ylmethyl)stilbene-4-carbonitrile, (Z)-α-(1,2,4-triazol-1-ylmethyl)-4'-(trifluoromethyl)stilbene-4-carbonitrile, (E)-β-fluoro-α-(1,2,4-triazol-1-ylmethyl)stilbene-4,4'-dicarbonitrile, (Z)-4'-fluoro-α-(imidazol-1-yl-methyl)stilbene-4-carbonitrile, (Z)-2',4'-dichloro-α-(imidazol-1-ylmethyl)stilbene-4-carbonitrile, (Z)-4'-chloro-α-(imidazol-1-ylmethyl)stilbene-4-carbonitrile, (Z)-α-(imidazol-1-ylmethyl)stilbene4,4'-dicarbonitrile,(Z)-α-(5-methylimidazol-1-ylmethyl)stilbene-4,4'-dicarbonitrile, and (Z)-2-[2-(4-cyanophenyl)3-(1,2,4-triazol-1-yl)propenyl]pyridine-5-carbonitrile as disclosed in European Patnet Application, Publication No. 299,683; (1R∗,2R∗)-6-fluoro-2-(4-fluorophenyl)-1,2,3,4-tetrahydro-1-(1H-1,2,4-triazol-1-ylmethyl)naphthalene, (1R∗,2R∗)-6-fluoro-2-(4-fluorophenyl)-1,2,3,4-tetrahydro-1-(1H-imidazolylmethyl)-naphthalene, (1R∗,2R∗)-and (1R∗,2S∗)-2-(4-fluorophenyl)-1,2,3,4-tetrahydro-1-(1H-1,2,4-triazol-1-yl-methyl)naphthalene-6-carbonitrile, (1R∗,2R∗)-and (1R∗,2S∗)-2-(4-fluorophenyl)-1,2,3,4-thetahydro-1-(1H-imidazolylmethyl)naphthalene-6-carbonitrile, (1R∗,2R∗)-and (1R∗,2S∗)-1,2,3,4-tetrahydro-1-(1H-1,2,4-triazol-1-ylmethyl)naphthalene-2,6-dicarbonitrile, (1R∗,2R∗)-and (1R∗,2S∗)-1,2,3,4-tetrahydro-1-(1H-imidazol-1-ylmethyl)naphthalene-2,6-dicarbonitrile, and(1R∗,2S∗)-2-(4-fluorophenyl)1,2,3,4-tetrahydro-1-(5-methyl-1H-imidazolylmethyl)naphthalene-6-carbonitrile as disclosed in European Patent Application, Publication No. 281,283; 8-chloro-5-(4-chlorophenyl)-5H-indeno[1,2-d] pyrimidine as disclosed in U.S. Patnet No. 4,769,378; 5-bis (4-chlorophenyl) methylpyrimidine as disclosed in U.S. Patent No. 4,762,836; 10-(2-propynyl)-estr-4-ene-3,17-dione as disclosed in U.S. Patent No. 4,322,416; 6-[(4-chlorophenyl) (1H-1,2,4-triazol-1-yl) methyl]-1-methyl-1H-benzotriazole as described in European Patent Application, Publication No. 293,978; 1-methylandrosta-1,4-dien-3,17-dione as disclosed in U.S. Patent No.4,591,585; 3-ethyl-3-(4-pyridyl)piperidine-2,6-dione as disclosed in British Patent GB 2,151,226; 4-hydroxyandrostene-3,17-dione as disclosed in U.S. Patent No. 4,500,523; 4-(5,6,7,8-tetrahydroimidazo[1,5-a]pyridin-5yl)benzonitrile as disclosed in U.S. Patent No. 4,728,645; 6-methyleneandrosta-1,4-diene-3,17-dione, 4-aminoadrostan-1,4,6-trien-3, 17-dione and 4-aminoandrosta-4,6-diene-3,17-dione as disclosed in U.S. Patent No. 4,757,061; 3-(1H-imidazol-1-ylmethyl)-2-methyl- 1H-indole-1-propanoic acid as disclosed in U.S. Patent No. 4,273,782; 5-[3-chlorophenyl)(1H-imidazol-1-yl)methyl]-1H-benzimidazole as disclosed in European Patent Application, Publication No. 260,744; 10β-thiiranylestr-4-ene-3,17-dione and 10β-oxiranylestr-4-ene-3, 17-dione as disclosed in J. Organ. Chem. 53: 5947-5951 (1988); 3-ethyl-3-(4-pyridyl)piperidine-2,6-dione as described in U.S. Patent No. 4,668,689; 3-(4-aminophenyl)3-ethylpyrrolidine-2,5-dione as disclosed in J. Med. Chem. 29: 520-523 (1986); 1-(7-carboxyheptyl)-imidazole as described in U.S. Patent No. 4,320,134; 1,1-dimethyl-8-(1H-1,2,4-triazol-1-ylmethyl)-2 (1H)-naphtho [2,1-b]furanone (1a) as disclosed in European Patent Application, Publication No. 316,097; ± 5- (p-cyanophenyl)-5, 6, 7, 8-tetrahydroimidazo-[1, 5-a] pyridine hydrochloride as disclosed in U.S. Patent No. 4,617,307; 1,4,6-androstatriene-3,17-dione disclosed in Biochem. Pharmac. 31: 2017-2023 (1982) and other compounds well known in the art of aromatase inhibition and cancer therapy such as bis-(p-cyanophenyl)-imidazo-1-yl-methane hemisuccinate and pharmaceutically acceptable derivatives, acid addition salts and possible stereochemically isomeric forms thereof, if and where appropriate. The invention is also intended to include any biologically active equivalents of an aromatase inhibitor as described above.

Fish or shellfish eggs such as those from Tilapia, *Oreochromis niloticus*, for example, are obtained through artificial fertilization and incubation by the techniques of; Rothbard and Pruginin, Aquaculture 5:315-321( 1975) and Rothbard and Hulata, Prog. Fish-Cult. 42:203-204(1980). Fry are obtained either from spawning females after about 16 days of buccal incubation, or from incubators from about 10 to about 14 days post-fertilization.

The aromatase inhibitor or inhibitors are dissolved or suspended in a physiologically acceptable carrier. Such aqueous physiologically acceptable carriers include, but are not limited to water, saline, physiologic saline, buffer saline, phosphate buffered saline, phosphate buffered saline glucose. Non-aqueous physiologically acceptable carriers include, but are not limited to, 1,2-propane-diol, dimethyl sulfoxide, ethanol and the like. These non-aqueous carriers are then used to dissolve the aromatase inhibitor in an entirely aqueous solution. The aromatase inhibitors can be initially diluted in an aqueous or non-aqueous acceptable carrier to a concentration of from about 1 μg/ml to about 100 μg/ml. This can be further diluted to a final treatment concentration which is added to the water in which the fish or shellfish are being maintained. The treatment concentration is form about 10 μg/L to about 1000 μg/L. It is to be understood that the concentration may very depending upon the type of fish or shellfish that is being treated and one skilled in the art could determine the required amount without undue experimentation.

Aromatase inhibitor treatment by immersion of fertilised eggs is carried out in appropriate containers under conditions that allow the normal development of the fertilized embryos, these conditions are well known in the art. Periods of immersion range from approximately two hours to approximately seven weeks. Immersion can begin with eggs at approximately the eyed-egg stage through to, and including, the period of sexual differentiation.

Hatched fish or fry and hatched shellfish capable of consumming whole food can be treated by administering the aromatase inhibitor to the normal food. The aromatase inhibitor is added to commercial fish diets by evaporation from ethanol, Guerrero, Trans. Am. Fish. Soc. 104:342-348(1975). Approximately 40 milligrams of aromatase inhibitor are added per kilogram of food and the fry are fed approximately twice daily from the time of first feeding onward. At approximately 3 months of age when the fish have reached sexual maturity, phenotypic sex and genetic sex are determined by the means well known in the art. This may include visual inspection, size measurements, gonadal squashes, measurement of serum marker proteins, and breeding experiments. The conversion of the genotypic female fish or shellfish to the phenotypic male fish or shellfish results in secondary sex characteristics such as increased weight gain and increased feed efficiency associated with male phenotype.

A major advantage of treating fish and shellfish with active aromatase inhibitors is the inhibition of the conversion of androgens to estrogens which normally takes place in developing female fish. This inhibition results in an increase in the ratio of androgens to estrogens which induces or, converts the embryo to, the male phenotype without having to treat the embryos with androgens or other hormones.

In many species of fish there is a direct relationship between body size and reproduction capacity. In general, growth rates decrease as the fish reaches sexual maturity as evidenced by gonadal maturation. This relationship is complex with the gonadal maturation and somatic growth each being controlled by several different growth factors. However, delayed sexual maturation resulting form the processes described above should provide for an extended period of growth. This delayed sexual maturation can be achieved either through generation of imperfect gonads or through the production of a monosex populations of fish. This growth, unrestricted by sexual differentiation, will result in enhanced economic gains for aquaculture of fish. It is anticipated that the same will apply to shellfish.

## EXAMPLE

Fish eggs such as those from Tilapia, *Oreochromis niloticus* are obtained through artificial fertilization and incubation by the techniques of; Rothbard and Pruginin, Aquaculture 5:315-321(1975) and Rothbard and Hulata, Prog. Fish-Cult. 42:203-204(1980). The aromatase inhibitor is dissolved or suspended in water at final concentrations of from 10 μg/ml to about 1000 μg/ml. Fertilised eggs, the eyed-egg stage or later developed stages, are immersed in the aromatase inhibitor solution for periods of approximately two hours to approximately seven weeks. Following aromatase inhibitor treatement the eggs are removed and placed in rearing enviornments known in the art so that the eggs will hatch. At various times after treatment the embryos or fish are evaluated to determine sex and ultamitely the conversion of genotypically female fish into phenotypically male fish as discussed below.

Fish fry are obtained either from spawning females after about 16 days of buccal incubation, or from incubators from about 10 to about 14 days post-fertilization and are subjected to treatment with an aromatase inhibitor or inhibitors as described above.

Hatched fish or fry capable of consumming whole food are treated by administering the aromatase inhibitor to the normal food. The aromatase inhibitor is added to commercial fish diets by evaporation from ethanol, Guerrero, Trans. Am. Fish. Soc. 104:342-348( 1975). Approximately 40 milligrams of aromatase inhibitor are added per kilogram of food and the fry are fed approximately twice daily from the time of first feeding onward. At approximately 3 months of age when the fish have reached sexual maturity, phenotypic sex and genetic sex are determined by standard means. This includes visual inspection, size measurements, gonadal squashes, measurement of serum marker proteins, or breeding experiments. The conversion of the genotypic female fish to the phenotypic male fish results in secondary sex characteristics such as increased weight gain and increased feed efficiency associated with male phenotype.

## Claims

1. A method for converting genotypic female fish into phenotypic male fish comprising administering to a healthy fish egg, embryo or fry a phenotypic converting amount of a substance which prevents the conversion of androgens to estrogens and results in the conversion of genotypic female fish into phenotypic male fish.

2. A method for converting genotypic female shellfish into phenotypic male shellfish comprising administering to a healthy shellfish egg, embryo or fry a phenotypic converting amount of a substance which prevents the conversion of androgens to estrogens and results in the conversion of genotypic female shellfish into phenotypic male shellfish.

3. A method for converting genotypic female fish into phenotypic male fish comprising administering to a healthy fish egg, embryo or fry a phenotypic converting amount of a substance which prevents the conversion of testosterone or androstenedione to estradiol or estrone and results in the conversion of genotypic female fish into phenotypic male fish.

4. A method for converting genotypic female shellfish into phenotypic male shellfish comprising administering to a healthy shellfish egg, embryo or fry a phenotypic converting amount of a substance which prevents the conversion of testosterone or androstenedione to estradiol or estrone and results in the conversion of genotypic female shellfish into phenotypic male shellfish.

5. A method for converting genotypic female fish or shellfish into phenotypic male fish or shellfish comprising administering to a healthy fish or shellfish egg, embryo or fry a phenotypic converting amount a an aromatase inhibitor.

6. The aromatase inhibitors of claim 5 wherein the said aromatase inhibitor is a steroid substrate analogue or a non-steroid analogue which binds and inactivates aromatase or is a compound that inhibits the cytochrome P450 component of the aromatase complex.

7. The aromatase inhibitor of claim 5 wherein the said aromatase inhibitor is selected from the group consisting of: 6-[(1$\underline{H}$-imidazol-1-yl)phenylmethyl]-1 methyl-1$\underline{H}$-benzotriazole, 6-[(4-chlorophenyl)(1$\underline{H}$-1,2,4-triazol-1-yl)methyl]-1-methyl-1$\underline{H}$-benzotriazole as described in Publication No. 293,978; 2,2-[5-(1$\underline{H}$-1,2,4,-triazol-1-ylmethyl)-1,3-phenylene]di(2-methylpropionitrile), 2,2-[5-(imidazol-1-ylmethyl)-1,3-phenylene]-di(2-methylpropionitrile), 2-[3-(1-hydroxy-1methylethyl)-5-(1$\underline{H}$-1,2,4-triazol-1-ylmethylphenyl]-2-methylpropionitrile, and 2,2-[5-dideuterio(1$\underline{H}$-1,2,4-triazol-1-yl)methyl-1,3-phenylene]di(2trideuteriomethyl-3,3,3-(trideuteriopropiononitrile), and 2,2-[5-dideuterio(1$\underline{H}$-1,2,4-triazol-1-ylmethyl-1,3-phenylene)di(2-methylpropiononitrile); 1,1-dimethyl-8-(1$\underline{H}$-1,2,4-triazol-1-ylmethyl)2-(1$\underline{H}$)-naphtho[2,1-b] furanone, 1,2-dihydro-1,1-dimethyl-2-oxo-8-(1$\underline{H}$-1,2,4-triazol-1-ylmethyl)naphtho-[2,1-b]furan-7-carbonitrile, 1,2-dihydro-1,1-dimethyl-2-oxo-8-(1$\underline{H}$-1,2,4-triazol-1-ylmethyl)naphtho[2,1-b]furan-7-carboxamide, and 1,2dihydro-1,1-dimethyl-2-oxo-8-[di(1H-1,2,4-triazol-1 yl)methyl]naphtho[2,1-b]furan-7-carbonitrile; 2-(4-chlorobenzyl)-2-fluoro-1,3-di(1,2,4-triazol-1-yl)propane, 2-fluoro-2-(2-fluoro-4-chlorobenzyl)-1,3-di(1,2,4-triazol-1-yl)propane, 2-fluoro-2-(2-fluoro-4-trifluoromethylbenzyl)-1,3-di(1,2,4-triazol-1-yl-)propane, 3-(4-chlorophenyl)-1-(1,2,4-triazol-1-yl)-2-(1,2,4-triazol-1-ylmethyl)butan-2-ol, 2-(4-chloro-α-fluorobenzyl)1,3-di(1,2,4-triazol-1-yl)propan-2-ol, 2-(4-chlorobenzyl)1,3-bis(1,2,4-triazol-1-yl)propane, 4-[2-(4-chlorophenyl)-1,3-di(1,2,4-triazol-1-ylmethyl)ethoxymethyl]-benzonitrile, 1-(4-fluorobenzyl)-2-(2-fluoro-4-trifluoromethylphenyl)-1,3-di(1,2,4-triazol-1-yl)propan-2-ol, 2-(4-chlorophenyl)-1-(4-fluorophenoxy)-1,3-di(1,2,4-triazol-1-yl)propan-2-ol, 1-(4-cyanobenzyl)2-(2,4-difluorophenyl)-1,3-di(1,2,4-triazol-1-yl)-propan-2-ol and 2-(4-chlorophenyl)1-phenyl-1,3-di(1,2,4-triazol-1-yl)-propan-2-ol; 5-bis(4-chlorophenyl)methylpyrimidine; α,α-bis(4-chlorophenyl)-2-pyrazinemethanol; N-(2,4-difluorophenyl)-N-benzyl-3-pyridinemethanamine and N-(2-chlorophenyl-α-(4-fluorophenyl)-3-pyridinemethanamine; 1-(10,11-dihydro-5H-dibenzo[a,d]cyclohepten-5-yl)- 1H-imidazole and 1-(9H-fluoren-9-yl)-1H-imidazole as disclosed in U.S. Patent No. 4,757,082; 3-bis(4-chlorophenyl)-3-methylpyridine and α,α-bis(4-chlorophenyl)-3-pyridinemethanol; 5H-5-(4-cyanophenyl)-6,7-dihydropyrrolo[1,2-c]imidazole and 5H-5-(4-cyanophenyl)-6,7,8,9-tetrahydroimidazo[1,5-a]azepine; 5-[(1 $\underline{H}$-imidazol-1-yl)phenylmethyl]-2-methyl-1 $\underline{H}$-benzimidazole and 5-[(3-chlorophenyl)(1 $\underline{H}$-imidazol-1-yl)-methyl]-1 $\underline{H}$-benzimidazole; ($\underline{Z}$)-α-(1,2,4-triazol-1-ylmethyl)stilbene-4,4′-dicarbonitrile, ($\underline{Z}$)-4′-chloro-α-(1,2,4-triazol-1-ylmethyl)stilbene-4-carbonitrile, ($\underline{Z}$)-α-(1,2,4-triazol-1-ylmethyl)-4′-(trifluoromethyl)stilbene-4-carbonitrile, ($\underline{E}$)-β-fluoro-α-(1,2,4-triazol-1-ylmethyl)stilbene-4,4′-dicarbonitrile, ($\underline{Z}$)-4′-fluoro-α-(imidazol-1-ylmethyl)stilbene-4-carbonitrile, ($\underline{Z}$)-2′,4′-dichloro-α-(imidazol-1-ylmethyl)stilbene-4-carbonitrile, ($\underline{Z}$)-4′-chloro-α-(imidazol-1-ylmethyl)stilbene-4-carbonitrile, ($\underline{Z}$)-α-(imidazol-1-ylmethyl)stilbene-4,4′-dicarbonitrile,($\underline{Z}$)-α-(5-methylimidazol-1-ylmethyl)stilbene-4,4′-dicarbonitrile, and (Z)-2-[2-(4-cyanophenyl)-3-(1,2,4-triazol-1-yl)propenyl]pyridine-5-carbonitrile; (1R∗ ,2R∗)-6-fluoro-2-(4-fluorophenyl)-1,2,3,4-tetrahydro-1-(1$\underline{H}$-1,2,4-triazol-1-ylmethyl)naphthalene, (1R∗ ,2R∗)-6-fluoro-2-(4-fluorophenyl) 1,2,3,4-tetrahydro-1-(1$\underline{H}$-imidazolyl-methyl)-naphthalene, (1R∗ ,2R∗)-and (1R∗,2S∗)-2-(4-fluorophenyl)-1,2,3,4-tetrahydro-1-(1$\underline{H}$-1,2,4-triazol-1-ylmethyl)naphthalene-6-carbonitrile, (1R∗ ,2R∗)-and (1R∗ ,2S∗)-2-(4-fluorophenyl)-1,2,3,4-thetahydro-1-(1$\underline{H}$-imidazolylmethyl)naphthalene-6-carbonitrile, (1R∗ ,2R∗)-and (1R∗ ,2S∗)-1,2,3,4-tetrahydro-1-(1H-1,2,4-triazol-1-ylmethyl)naphthalene-2,6-dicarbonitrile, (1R∗ ,2R∗)-and (1R∗ ,2S∗)-1,2,3,4-tetrahydro-1-(1H-imidazol-1-ylmethyl)naphthalene-2,6-dicarbonitrile, and(1R∗ ,2S∗)-2-(4-fluorophenyl)1,2,3,4-tetrahydro-1-(5-methyl-1H-imidazolylmethyl)naphthalene-6-carbonitrile; 8-chloro-5-(4-chlorophenyl)-5H-indeno[1,2-d] pyrimidine; 5-bis (4-chlorophenyl) methylpyrimidine; 10-(2-propynyl)-estr-4-ene-3,17-dione as disclosed in U.S. Patent No. 4,322,416; 6-[(4-chlorophenyl) (1H-1,2,4-triazol-1-yl) methyl]-1-methyl-1H-benzotriazole; 1-methylandrosta-1,4-dien-3,17-dione; 3-ethyl-3-(4-pyridyl)piperidine-2,6-dione; 4-hydroxyandrostene-3,17-dione; 4-(5,6,7,8-tetrahydroimidazo[1,5-a]pyridin-5yl)benzonitrile; 6-methyleneandrosta-1,4-diene-3,17-dione, 4-aminoadrostan-1,4,6-trien-3,17-dione and 4-aminoandrosta-4,6-diene-3,17-dione; 3-(1H-imidazol-1-ylmethyl)2-methyl-1H-indole-1-propanoic acid as disclosed in U.S. Patent No. 4,273,782; 5-[3-chlorophenyl)(1H-imidazol-1-yl)methyl]-1H-benzimidazole; 10β-thiiranylestr-4-ene-3,17-dione and 10β-oxiranylestr-4-ene-3,17-dione; 3-ethyl-3-(4-pyridyl)piperidine-2,6-dione; 3-(4-aminophenyl)-3-ethyl-pyrrolidine-2,5-dione; 1-(7-carboxyheptyl)-imidazole; 1,1-dimethyl-8-(1H-1,2,4-triazol-1-ylmethyl)2 (1H)-naphtho [2,1-b]furanone (1a); ± 5- (p-cyanophenyl)-5, 6, 7, 8-tetrahydroimidazo [1,5-a] pyridine hydrochloride; 1,4,6-androstatriene-3,17-dione; and bis-(p-cyanophenyl)-imidazo-1-yl-methane hemisuccinate and pharmaceutically acceptable deritives, acid addition salts thereof.

8. The aromatase inhibitor of claim 7 wherein said aromatase inhibitor is ± 5-(p-cyanophenyl)-5,6,7,8-tetrahydroimidazo[1,5-α] pyridine hydrochloride.

9. The aromatase inhibitor of claim 5 wherein said aromatase inhibitor is 1,4,6-androstatriene-3,17-dione.

10. The method of claim 1 wherein the fish or shellfish embryos are treated with one or more aromatase inhibitors.

11. Phenotypically male fish or shellfish comprising genotypic females treated with one or more aromatase inhibitor which converts the females to a male phenotype.

12. Genotypically female fish or shellfish having a male phenotype resulting from treating the developing embryo with one or more aromatase inhibitor.

13. A composition for converting genotypic female fish or shellfish into phenotypic male fish or shellfish comprising administering to a healthy fish or shellfish egg, embryo or fry a phenotypic converting amount of a substance which prevents the conversion of androgens to estrogens and resets in the conversion of genotypic female fish or shellfish into phenotypic male fish or shellfish.

14. The composition for converting genotypic female fish or shellfish into phenotypic males of claim 13 wherein the phenotypic converting substance is one or more aromatase inhibitors in a physiologically acceptable carrier.

15. The aromatase inhibitor of claim 14 wherein the said aromatase inhibitor is a steroid substrate analogue or a non-steroid analogue which binds and inactivates the enzyme or a compound that inhibits the cytochrome P450 component of the enzymatic activity.

16. The aromatase inhibitor of claim 14 wherein the said aromatase inhibitor is selected from the group consisting of: 6-[(1H-imidazol-1-yl)phenylmethyl]-1 methyl-1H-benzotriazole, 6-[(4-chlorophenyl)(1H-1,2,4-triazol-1-yl)methyl]-1-methyl-1H-benzotriazole as described in Publication No. 293,978; 2,2-[5-(1H-1,2,4,-triazol-1-ylmethyl)-1,3-phenylene]di(2-methylpropiononitrile), 2,2-[5-(imidazol-1-ylmethyl)-1,3-phenylene]-di(2-methylpropiononitrile), 2-[3-(1-hydroxy-1-methylethyl)-5-(1H-1,2,4-triazol-1-ylmethylphenyl]-2-methylpropiononitrile, and 2,2-[5-dideuterio(1H-1,2,4-triazol-1-yl)methyl-1,3-phenylene]di(2-trideuteriomethyl-3,3,3-(trideuteriopropiononitrile), and 2,2-[5-dideuterio(1H-1,2,4-triazol-1-ylmethyl-1,3-phenylene)di(2-methylpropiononitrile); 1,1-dimethyl-8-(1H-1,2,4-triazol-1-ylmethyl)-2(1H)-naphtho[2,1-b] furanone, 1,2-dihydro-1,1-dimethyl-2-oxo-8-(1H-1,2,4-triazol-1-ylmethyl)naphtho-[2,1-b]furan-7-carbonitrile, 1,2-dihydro-1,1-dimethyl-2-oxo-8-(1H-1,2,4-triazol-1-ylmethyl)-naphtho[2,1-b]furan-7-carboxamide, and 1,2-dihydro-1,1-dimethyl-2-oxo-8-[di(1H-1,2,4-triazol-1 yl)methyl]naphtho[2,1-b]furan-7-carbonitrile; 2-(4-chlorobenzyl)-2-fluoro-1,3-di(1,2,4-triazol-1-yl)propane, 2-fluoro-2-(2-fluoro-4-chlorobenzyl)-1,3-di(1,2,4-triazol-1-yl)propane, 2-fluoro-2-(2-fluoro-4-trifluoromethylbenzyl)-1,3-di(1,2,4-triazol-1-yl)propane, 3-(4-chlorophenyl)-1-(1,2,4-triazol-1-yl)2-(1,2,4-triazol-1-ylmethyl)butan-2-ol, 2-(4-chloro-α-fluorobenzyl)1,3-di(1,2,4-triazol-1-yl)propan-2-ol, 2-(4-chlorobenzyl)1,3-bis(1,2,4-triazol-1-yl)propane, 4-[2-(4-chorophenyl)-1,3-di(1,2,4-triazol-1-ylmethyl)ethoxymethyl]-benzonitrile, 1-(4-fluorobenzyl)-2-(2-fluoro-4-trifluoromethylphenyl)-1,3-di(1,2,4-triazol-1-yl)propan-2-ol, 2-(4-chlorophenyl)-1-(4-fluorophenoxy)-1,3-di(1,2,4-triazol-1-yl)propan-2-ol, 1-(4-cyanobenzyl)2-(2,4-difluorophenyl)-1,3-di(1,2,4-triazol-1-yl)-propan-2-ol and 2-(4-chlorophenyl)1-phenyl-1,3-di(1,2,4-triazol-1-yl)-propan-2-ol; 5-bis(4-chlorophenyl)methylpyrimidine; α,α-bis(4-chlorophenyl)-2-pyrazinemethanol; N-(2,4-difluorophenyl)-N-benzyl-3-pyridinemethanamine and N-(2-chlorophenyl-α-(4-fluorophenyl)-3-pyridinemethanamine; 1-(10,11-dihydro-5H-dibenzo[a,d]cyclohepten-5-yl)-1H-imidazole and 1-(9H-fluoren-9-yl)-1H-imidazole as disclosed in U.S. Patent No. 4,757,082; 3-bis(4-chlorophenyl)-3-methylpyridine and α,α-bis(4-chlorophenyl)-3-pyridinemethanol; 5H-5-(4-cyanophenyl)-6,7-dihydropyrrolo[1,2-c]imidazole and 5H-5-(4-cyanophenyl)-6,7,8,9-tetrahydroimidazo[1,5-a]azepine; 5-[(1 H-imidazol-1-yl)phenylmethyl]-2-methyl-1 H-benzimidazole and 5-[(3-chlorophenyl)(1 H-imidazol-1-yl)-methyl]-1 H-benzimidazole; (Z)-α-(1,2,4-triazol-1-ylmethyl)stilbene-4,4'-dicarbonitrile, (Z)-4'-chloro-α-(1,2,4-triazol-1-ylmethyl)stilbene-4-carbonitrile, (Z)-α-(1,2,4-triazol-1-ylmethyl)-4'-(trifluoromethyl)stilbene-4-carbonitrile, (E)-β-fluoro-α-(1,2,4-triazol-1-ylmethyl)stilbene-4,4'-dicarbonitrile, (Z)-4'-fluoro-α-(imidazol-1-ylmethyl)stilbene-4-carbonitrile, (Z)-2',4'-dichloro-α-(imidazol-1-ylmethyl)stilbene-4-carbonitrile, (Z)-4'-chloro-α-(imidazol-1-ylmethyl)stilbene-4-carbonitrile, (Z)-α-(imidazol-1-yl-methyl)stilbene-4,4'-dicarbonitrile,(Z)-α-(5-methylimidazol-1-ylmethyl)stilbene-4,4'-dicarbonitrile, and (Z)-2-[2-(4-cyanophenyl)-3-(1,2,4-triazol-1-yl)propenyl]pyridine-5-carbonitrile; (1R∗,2R∗)-6-fluoro-2-(4-fluorophenyl)-1,2,3,4-tetrahydro-1-(1H-1,2,4-triazol-1-ylmethyl)naphthalene, (1R∗,2R∗)-6-fluoro-2-(4-fluorophenyl)-1,2,3,4-tetrahydro-1-(1H-imidazolymethyl)-naphthalene, (1R∗,2R∗)and (1R∗,2S∗)2-(4-fluorophenyl)-1,2,3,4-tetrahydro-1-(1H-1,2,4-triazol-1-ylmethyl)naphthalene-6-carbonitrile, (1R∗,2R∗)-and (1R∗,2S∗)-2-(4-fluorophenyl)-1,2,3,4-thetahydro-1-(1H-imidazolylmethyl)naphthalene-6-carbonitrile, (1R∗,2R∗)-and (1R∗,2S∗)-1,2,3,4-tetrahydro-1-(1H-1,2,4-triazol-1-ylmethyl)naphthalene-2,6-dicarbonitrile, (1R∗,2R∗)-and (1R∗,2R∗)-1,2,3,4-tetrahydro-1-(1H-imidazol-1-ylmethyl)naphthalene-2,6-dicarbonitrile, and(1R∗,2S∗)-2-(4-fluorophenyl)1,2,3,4-tetrahydro-1-(5-methyl-1H-imidazolylmethyl)naphthalene-6-carbonitrile; 8-chloro-5-(4-chlorophenyl)-5H-indeno[1,2-d] pyrimidine; 5-bis (4-chlorophenyl) methylpyrimidine; 10-(2-propynyl)-estr-4-ene-3,17-dione as disclosed in U.S. Patent No. 4,322,416; 6-[(4-chlorophenyl) (1H-1,2,4-triazol-1-yl) methyl]-1-methyl-1H-benzotriazole; 1-methylandrosta-1,4-dien-3, 17-dione; 3-ethyl-3-(4-pyridyl)piperidine-2,6-dione; 4-hydroxyandrostene-3,17-dione; 4-(5,6,7,8-tetrahydroimidazo[1,5-a]pyridin-5yl)benzonitrile; 6-methyleneandrosta-1,4-diene-3,17-dione, 4-aminoadrostan-1,4,6-trien-3,17-dione and 4-aminoandrosta-4,6-diene-3, 17-dione; 3-(1H-imidazol-1-ylmethyl)-2-methyl- 1H-indole-1-propanoic acid as disclosed in U.S. Patent No. 4,273,782; 5-[3-chlorophenyl)(1H-imidazol-1-yl)methyl]-1H-benzimidazole ; 10β-thiiranylestr-4-ene-3,17-dione and 10β-oxiranylestr-4-ene-3,17-dione; 3-ethyl-3-(4-pyridyl)piperidine-2,6-dione; 3-(4-aminophenyl)-3-ethyl-pyrrolidine-2,5-dione; 1-(7-carboxyheptyl)-imidazole; 1,1-dimethyl-8-(1H-1,2,4-triazol-1-ylmethyl)-2 ( 1H)naphtho [2,1-b]furanone (1a); ± 5-(p-cyanophenyl)-5, 6, 7, 8-tetrahydroimidazo [1, 5-a] pyridine hydrochloride; 1,4,6-androstatriene-3,17-dione; and bis-(p-cyanophenyl)-imidazo-1-yl-methane hemisuccinate and pharmaceutically acceptable deritives, acid addition salts thereof.

17. The aromatose inhibitor of claim 16 wherein said aromatase inhibitor is ± 5- (p-cyanophenyl)5, 6, 7, 8-tetrahydroimidazo[1,5-α] pyridine hydrochloride.

18. The aromatase inhibitor of claim 16 wherein said aromatase inhibitor is 1,4,6-androstatriene-3,17-dione.

19. A method for increasing weight gain and or feed efficiency in genotypic female fish or shellfish comprising treatment of geontypic female fish or shellfish with one or more aromatase inhibitors.

20. The aromatase inhibitor of claim 19 wherein the said aromatase inhibitor is selected from the group consisting of: 6-[(1H-imidazol-1-yl)phenylmethyl]-1 methyl-1H-benzotriazole, 6-[(4-chlorophenyl)(1H-1,2,4-triazol-1-yl)methyl]-1-methyl-1H-benzotriazole as described in Publication No. 293,978; 2,2-[5-(1H-1,2,4,-triazol-1-ylmethyl)-1,3-phenylene]di(2-methylpropiononitrile), 2,2-[5-(imidazol-1-ylmethyl)1,3-phenylene]-di(2-methylpropiononitrile), 2-[3-(1-hydroxy-1-methylethyl)-5-(1H-1,2,4-triazol-1-ylmethylphenyl]-2-methylpropiononitrile, and 2,2-[5-dideuterio(1H-1,2,4-triazol-1-yl)methyl-1,3-phenylene]di(2-trideuteriomethyl-3,3,3-(trideuteriopropiononitrile), and 2,2-[5-dideuterio(1H-1,2,4-triazol-1-ylmethyl-1,3-phenylene)di(2-methylpropiononitrile); 1,1-dimethyl-8-(1H-1,2,4-triazol-1-ylmethyl)-2(1H)-naphtho[2,1-b] furanone, 1,2-dihydro-1,1-dimethyl-2-oxo-8-(1H-1,2,4-triazol-1-ylmethyl)naphtho-[2,1-b]furan-7-carbonitrile, 1,2-dihydro-1,1-dimethyl-2-oxo-8-(1H-1,2,4-triazol-1-ylmethyl)-naphtho[2,1-b]furan-7-carboxamide, and 1,2-dihydro-1,1-dimethyl-2-oxo-8-[di(1H-1,2,4-triazol-1 yl)methyl]naphtho[2,1-b]furan-7-carbonitrile; 2-(4-chlorobenzyl)-2-fluoro-1,3-di(1,2,4-triazol-1-yl)propane, 2-fluoro-2-(2-fluoro-4-chlorobenzyl)1,3-di(1,2,4-triazol-1-yl)propane, 2-fluoro2-(2-fluoro-4-trifluoromethylbenzyl)-1,3-di(1,2,4-triazol-1-yl)propane, 3-(4-chlorophenyl)1-(1,2,4-triazol-1-yl)-2-(1,2,4-triazol-1-ylmethyl)butan-2-ol, 2-(4-chloro-α-fluorobenzyl)1,3-di(1,2,4-triazol-1-yl)propan-2-ol, 2-(4-chlorobenzyl)1,3-bis(1,2,4-triazol-1-yl)propane, 4-[2-(4-chlorophenyl)-1,3-di(1,2,4-triazol-1-ylmethyl)ethoxymethyl]-benzonitrile,1-(4-fluorobenzyl)-2-(2-fluoro-4-trifluoromethylphenyl)-1,3-di (1,2,4-triazol-1-yl)propan-2-ol, 2-(4-chlorophenyl)-1-(4-fluorophenoxy)-1,3-di(1,2,4-triazol-1-yl)propan-2-ol, 1-(4-cyanobenzyl)2-(2,4-difluorophenyl)-1,3-di(1,2,4-triazol-1-yl)-propan-2-ol and 2-(4-chlorophenyl)1-phenyl-1,3-di(1,2,4-triazol-1-yl)-propan-2-ol; 5-bis(4-chlorophenyl)methylpyrimidine; α,α-bis(4-chlorophenyl)-2-pyrazinemethanol; N-(2,4-difluorophenyl)-N-benzyl-3-pyridinemethanamine and N-(2-chlorophenyl-α-(4-fluorophenyl)-3-pyridinemethanamine; 1-(10,11-dihydro-5H-dibenzo[a,d]cyclohepten-5-yl)-1H-imidazole and 1-(9H-fluoren-9-yl)-1H-imidazole as disclosed in U.S. Patent No. 4,757,082; 3-bis(4-chlorophenyl)-3-methylpyridine and α,α-bis(4-chlorophenyl)-3-pyridinemethanol; 5H-5-(4-cyanophenyl)-6,7-dihydropyrrolo[1,2-c]imidazole and 5H-5-(4-cyanophenyl)6,7,8,9-tetrahydroimidazo[1,5-a]azepine; 5-[(1 H-imidazol-1-yl)phenylmethyl]-2-methyl-1 H-benzimidazole and 5-[(3-chlorophenyl)(1 H-imidazol-1-yl)-methyl]-1 H-benzimidazole; (Z)-α-(1,2,4-triazol-1-ylmethyl)stilbene-4,4'-dicarbonitrile, (Z)-4'-chloro-α-( 1,2,4-triazol-1-ylmethyl)stilbene-4-carbonitrile, (Z)-α-(1,2,4-triazol-1-ylmethyl)-4'-(trifluoromethyl)stilbene-4-carbonitrile, (E)-β-fluoro-α-(1,2,4-triazol-1-ylmethyl)stilbene-4,4'-dicarbonitrile, (Z)-4'-fluoro-α-(imidazol- 1-ylmethyl)stilbene-4-carbonitrile, (Z)-2',4'-dichloro-α-(imidazol-1-ylmethyl)stilbene-4-carbonitrile, (Z)-4'-chloro-α-(imidazol-1-ylmethyl)stilbene-4-carbonitrile, (Z)-α-(imidazol-1-ylmethyl)stilbene-4,4'-dicarbonitrile,(Z)-α-(5-methylimidazol-1-yl-methyl)stilbene-4,4'-dicarbonitrile, and (Z)-2-[2-(4-cyanophenyl)-3-(1,2,4-triazol-1-yl)propenyl]pyridine-5-carbonitrile; (1R∗,2R∗)-6-fluoro-2-(4-fluorophenyl)-1,2,3,4-tetrahydro-1-(1H-1,2,4-triazol-1-ylmethyl)naphthalene, (1R∗ ,2R∗)6-fluoro-2-(4-fluorophenyl)-1,2,3,4-tetrahydro-1-(1H-imidazolylmethyl)-naphthalene, (1R∗ ,2R∗)-and (1R∗,2S∗)-2-(4-fluorophenyl)1,2,3,4-tetrahydro-1-(1H-1,2,4-triazol-1-ylmethyl)naphthalene-6-carbonitrile, (1R∗ ,2R∗)-and (1R∗ ,2S∗)-2-(4-fluorophenyl)-1,2,3,4-thetahydro-1-(1H-imidazolylmethyl)naphthalene-6-carbonitrile, (1R∗ ,2R∗)-and (1R∗ ,2S∗)-1,2,3,4-tetrahydro-1-(1H-1,2,4-triazol-1-ylmethyl)naphthalene-2,6-dicarbonitrile, (1R∗,2R∗)-and (1R∗,2S∗)-1,2,3,4-tetrahydro-1-(1H-imidazol-1-ylmethyl)naphthalene-2,6-dicarbonitrile, and(1R∗ ,2S∗)-2-(4-fluorophenyl)1,2,3,4-tetrahydro-1-(5-methyl-1H-imidazolylmethyl)naphthalene-6-carbonitrile; 8-chloro-5-(4-chlorophenyl)-5H-indeno[1,2-d] pyrimidine; 5-bis (4-chlorophenyl) methylpyrimidine; 10-(2-propynyl)-estr-4-ene-3,17-dione as disclosed in U.S. Patent No. 4,322,416; 6-[(4-chlorophenyl) (1H-1,2,4-triazol-1-yl) methyl]-1-methyl-1H-benzotriazole; 1-methylandrosta-1,4-dien-3,17-dione; 3-ethyl-3-(4-pyridyl)piperidine-2,6-dione; 4-hydroxyandrostene-3,17-dione; 4-(5,6,7,8-tetrahydroimidazo[1,5-a]pyridin-5yl)benzonitrile; 6-methyleneandrosta-1,4-diene-3,17-dione, 4-aminoadrostan-1,4,6-trien-3,17-dione and 4-aminoandrosta-4,6-diene-3,17-dione; 3-(1H-imidazol-1-ylmethyl)-2-methyl-1H-indole-1-propanoic acid as disclosed in U.S. Patent No. 4,273,782; 5-[3-chlorophenyl)(1H-imidazol-1-yl)methyl]-1H-benzimidazole; 10β-thiiranylestr-4-ene-3,17-dione and 10β-oxiranylestr-4-ene-3,17-dione; 3-ethyl-3-(4-pyridyl)piperidine-2,6-dione; 3-(4-aminophenyl)-3-ethyl-pyrrolidine-2,5-dione; 1-(7-carboxyheptyl)-imidazole; 1,1-dimethyl-8-(1H-1,2,4-triazol-1-ylmethyl)-2 ( 1H)-naphtho [2,1-b]furanone (1a); ± 5-(p-cyanophenyl)-5, 6, 7, 8-tetrahydroimidazo [1,5-a] pyridine hydrochloride; 1,4,6-androstatriene-3,17-dione; and bis-(p-cyanophenyl)-imidazo-1-yl-methane hemisuccinate and pharmaceutically acceptable deritives, acid addition salts thereof.

**21.** The aromatose inhibitor of claim 20 wherein said aromatase inhibitor is ± 5-(p-cyanophenyl)-5, 6, 7, 8-tetrahydroimidazo[1, 5-α] pyridine hydrochloride.

**22.** The aromatase inhibitor of claim 20 wherein said aromatase inhibitor is 1,4,6-androstatriene-3,17-dione.

European Patent Office

**EUROPEAN SEARCH REPORT**

Application Number

EP 92 30 3458

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | EP-A-0 433 084 (MERCK & CO. INC.) 19 June 1991<br><br>* claims 4-7 *<br>--- | 6-9,<br>13-18,<br>20-22 | A61K31/00<br>A61K31/44<br>A61K31/565 |
| X | J.FISH.BIOL.<br>vol. 26, no. 2, 1985,<br>pages 87 - 94;<br>D.J.MACINTOSH ET AL: 'Hormonal sex reversal of wild-spawned tilapia in India' | 11,12 | |
| Y | *abstract; pages 92-93: discussion*<br><br>--- | 1-5,10,<br>19 | |
| Y | PROC.NATL. ACAD.SCI<br>vol. 86, no. 10, 1989,<br>pages 3684 - 3688;<br>B.VIGIER ET AL.: 'Anti-Müllerian hormone produces endocrine sex reversal in fetal ovaries'<br>*page 3684, right column, paragraph 1*<br>* abstract *<br>--- | 1-5,10,<br>19 | |
| Y | PROC.NATL.SCI.COUNC.<br>vol. 4, no. 4, 1980,<br>pages 364 - 368;<br>CHIH-YUN HSU ET AL.: 'The effect of an Aromatase Inhibitor on Gonadal Sex Transformation in Female Tadpoles'<br>*Introduction*<br>* abstract *<br><br>----- | 1-5,10,<br>19 | TECHNICAL FIELDS SEARCHED (Int. Cl.5)<br><br>A61K |

The present search report has been drawn up for all claims

| Place of search | Date of completion of the search | Examiner |
|---|---|---|
| MUNICH | 03 SEPTEMBER 1992 | TZSCHOPPE D.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.............................................................................
& : member of the same patent family, corresponding document

EPO FORM 1503 03.82 (P0401)